(19)

Europäisches
Patentamt
European
Patent Office
Office européen
des brevets

(11) **EP 4 023 206 A1**

(12) **EUROPEAN PATENT APPLICATION**
published in accordance with Art. 153(4) EPC

(43) Date of publication:
**06.07.2022 Bulletin 2022/27**

(21) Application number: **20857853.4**

(22) Date of filing: **11.08.2020**

(51) International Patent Classification (IPC):
*A61K 8/19* (2006.01)          *A61K 8/37* (2006.01)
*A61K 8/34* (2006.01)          *A61K 8/04* (2006.01)
*A61Q 19/00* (2006.01)

(86) International application number:
**PCT/KR2020/010627**

(87) International publication number:
**WO 2021/040275 (04.03.2021 Gazette 2021/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.08.2019  KR 20190105752**

(71) Applicant: **Kolmar Korea Co., Ltd.
Sejong 30004 (KR)**

(72) Inventors:
• **KIM, Yong Woo
  Seoul 06800 (KR)**
• **KIM, Jin Young
  Seoul 07255 (KR)**
• **KYE, Sung Bong
  Yongin-si Gyeonggi-do 16964 (KR)**

(74) Representative: **Gulde & Partner
Patent- und Rechtsanwaltskanzlei mbB
Wallstraße 58/59
10179 Berlin (DE)**

(54) **DISPERSION OF CERIUM OXIDE FOR ENHANCING SKIN BARRIER, SKIN MOISTURIZING,
AND/OR BLOCKING FINE DUST, AND COSMETIC COMPOSITION COMPRISING SAME**

(57)    The prersen invention relates to a dispersion of cerium oxide showing excellent effects of enhancing the skin barrier, skin moisturizing and/or blocking fine dust, and a cosmetic composition comprising same. More specifically, the present invention provides a dispersion of cerium oxide that is a combination of cerium oxide and a specific dispersion medium and as such, can exhibit effects of moisturizing the skin and blocking fine dust while strengthening the skin barrier, and a cosmetic comprising the dispersion of cerium oxide as an active ingredient.

**EP 4 023 206 A1**

Processed by Luminess, 75001 PARIS (FR)

## Description

### Technical Field

The present disclosure relates to a cerium oxide dispersion for strengthening skin barrier, moisturizing skin, and/or blocking fine dust, and a cosmetic composition comprising the same.

### Background Art

The skin is the outermost layer that covers the outside of the living body and protects our body from external physical and chemical stimuli and is the most important organ that maintains homeostasis of the body from the external environment. This skin is composed of three layers from the outside: the epidermis, the dermis, and the subcutaneous layer.

Among those three layers, the epidermis is the outermost, and the stratum corneum, which is the upper layer of the epidermis, is composed of keratinocytes and intercellular lipids. The keratinocytes are keratinized cells that differentiate in the basal layer of the epidermis and are formed of a protein composed of keratin, and the intercellular lipid is formed of ceramide, cholesterol, fatty acids, and the like. Accordingly, the stratum corneum is formed in a unique lamellar liquid crystal structure, performs a skin barrier function, and prevents skin moisture loss.

In particular, ceramide is a key component that accounts for about 50% of intercellular lipid and corresponds to a major component that enables the lamellar structured stratum corneum to perform a skin barrier function.

Accordingly, as disclosed in Korean Patent No. 10-0435855, in general, in order to strengthen the skin barrier, various attempts have continued to prevent the lack or loss of ceramide, which is a major component in the keratin layer.

On the other hand, the function of the skin barrier may be reduced due to the lack or loss of components constituting the stratum corneum, as well as by external stimuli such as ultraviolet rays and fine dust. In particular, fine dust has the problem of weakening the skin barrier function by contaminating the skin itself and lowering the skin barrier function. In addition, fine dust has a problem of accelerating skin aging and skin moisture loss while weakening the skin barrier function by promoting collagen decomposition of the skin.

That is, there are various factors other than the lack or loss of the main component of the stratum corneum in the weakening of the skin barrier function. However, only a method to strengthen the skin barrier function by promoting the synthesis of ceramide and cholesterol, which are the main components of the stratum corneum, has been considered. However, resolving the shortage or loss of major components of the stratum corneum while protecting the skin from fine dust has not been considered at all.

Therefore, developing a cosmetic composition capable of enhancing the skin barrier function by protecting the skin from fine dust while preventing the shortage or loss of ceramide, which is a major component of the skin barrier, is required.

On the other hand, cerium oxide has been mainly used as a sunscreen agent, pigment, powder, etc., in cosmetic compositions up to now, and the utilization for other purposes was insignificant.

Therefore, in order to use cerium oxide for various purposes, the applicant completed the present disclosure by confirming that when cerium oxide is used with a specific combination of the dispersion medium, excellent ceramide synthesis promotion effects, moisturizing skin effects, fine dust blocking effects, and skin barrier enhancement effects can be implemented.

### Disclosure

### Technical Problem

A first objective of the present disclosure is to provide a cerium oxide dispersion that can implement a skin barrier strengthening effect by comprising cerium oxide with a dispersion medium of a specific combination.

A second objective of the present disclosure is to provide a cerium oxide dispersion that can implement a moisturizing skin effect by comprising cerium oxide with a dispersion medium of a specific combination.

A third objective of the present disclosure is to provide a cerium oxide dispersion that can implement fine dust blocking effect by comprising cerium oxide with a dispersion medium of a specific combination.

A fourth objective of the present disclosure is to provide a cosmetic composition capable of implementing the effect of strengthening the skin barrier, moisturizing the skin, and/or blocking fine dust by comprising the cerium oxide dispersion.

The objectives of the present disclosure are not limited to the technical problem as described above, and another technical problem may be derived from the following description.

**Technical Solution**

In order to achieve the first objective, the present disclosure provides a cerium oxide dispersion for strengthening a skin barrier, wherein the cerium oxide comprises: a cerium oxide; and a dispersion medium comprising an ester-based oil and a polyol, in which the cerium oxide and the dispersion medium are comprised in a weight ratio of 1:1 to 3.

In order to achieve the second objective, the present disclosure provides a cerium oxide dispersion for moisturizing the skin, wherein the cerium oxide comprises: a cerium oxide; and a dispersion medium comprising an ester-based oil and a polyol, in which the cerium oxide and the dispersion medium are comprised in a weight ratio of 1:1 to 3.

In order to achieve the third objective, the present disclosure provides a cerium oxide dispersion for blocking fine dust, wherein the cerium oxide comprises: a cerium oxide; and a dispersion medium comprising an ester-based oil and a polyol, in which the cerium oxide and the dispersion medium are comprised in a weight ratio of 1:1 to 3.

The cerium oxide may have an average particle diameter of 80 to 170 nanometers (nm).

The dispersion medium may comprise the ester-based oil and the polyol in a weight ratio of 1: 0.1 to 0.7.

The ester-based oil may comprise at least one selected from the group consisting of benzoate-based oil, adipate-based oil, carbonate-based oil, and caprylate-based oil.

The polyol may comprise at least one selected from the group consisting of glycerin, propylene glycol, dipropylene glycol, butylene glycol, and propanediol.

In order to achieve the fourth objective, the present disclosure provides a cosmetic composition for strengthening the skin barrier, moisturizing the skin, or blocking fine dust, comprising a cerium oxide dispersion for strengthening the skin barrier, moisturizing the skin, or blocking fine dust, wherein the cerium oxide comprises: a cerium oxide; and a dispersion medium comprising an ester-based oil and a polyol, in which the cerium oxide and the dispersion medium are comprised in a weight ratio of 1:1 to 3.

The cosmetic composition may comprise the cerium oxide dispersion in an amount of 0.1 to 30% by weight based on the total weight of the cosmetic composition.

The cosmetic composition may be any one formulation selected from the group consisting of a softening lotion, a nourishing lotion, an astringent lotion, a skin, lotion, an essence, a cream, a massage cream, a pack, a makeup base, BB cream, a foundation, a lipstick, a lip balm, a lip tint, a lip gloss, a sun cream, a sunscreen lotion, a sunscreen milk, a sunscreen stick, a cleansing foam, a cleansing cream, and cleansing water.

**Advantageous Effects**

The cerium oxide dispersion of the present disclosure can implement an effect of strengthening the skin barrier, moisturizing the skin, and/or blocking fine dust. In addition, in the present disclosure, cerium oxide is provided in the form of a dispersion, thereby improving a feeling of use such as skin application and skin adhesion.

The cosmetic composition of the present disclosure may enhance skin barrier, moisturize skin, and/or block fine dust as cerium oxide contains a dispersion dispersed in a specific combination of the dispersion medium. In addition, the cosmetic composition of the present disclosure comprises cerium oxide in the form of a dispersion, thereby having an excellent feeling of use.

**Best Mode**

Hereinafter, embodiments of the present disclosure will be described in detail with reference to the accompanying drawings so that those of ordinary skilled in the art to which the present disclosure pertains can easily carry out. However, the present disclosure may be embodied in several different forms and is not limited to the embodiments described herein. In order to clearly explain the present disclosure in the drawings, parts irrelevant to the description are omitted.

The terms or words used in the specification and claims of the present disclosure are not to be construed as limited in their ordinary or dictionary meanings, and the inventor appropriately defines the concept of the term in order to best describe his disclosure. It should be interpreted as meaning and concept consistent with the technical idea of the present disclosure based on the principle that it can be done.

Throughout the present specification, when a part "contains" a component, this means that other components may be further comprised rather than excluding other components unless otherwise opposed.

Throughout the specification of the present disclosure, "A and/or B" means A or B, or A and B.

Hereinafter, the present disclosure has been specifically described, but the present disclosure is not limited thereto.

The present disclosure provides a cerium oxide dispersion for strengthening the skin barrier, moisturizing the skin, and/or blocking fine dust.

In an embodiment of the present disclosure, the present disclosure provides a cerium oxide dispersion for strengthening the skin barrier, moisturizing the skin, and/or blocking fine dust, wherein the cerium oxide dispersion comprises: cerium oxide; and a dispersion medium comprising an ester-based oil and a polyol, in which the cerium oxide and the dispersion medium are comprised in a weight ratio of 1:1 to 3.

The cerium oxide dispersion of the present embodiment may simultaneously implement skin barrier strengthening, skin moisturizing, and fine dust blocking effects simultaneously. In addition, in the present disclosure, cerium oxide is provided in the form of a dispersion, thereby improving a feeling of use such as skin application and skin adhesion.

Cerium oxide is a white powder. The crystal structure of cerium oxide has a lanthanum oxide type structure belonging to the hexagonal crystal system. The cerium oxide was mainly used as a sunscreen previously , but in the present embodiment, effects of strengthening skin barriers, moisturizing skin, and/or blocking fine dust may be implemented by comprising the cerium oxide together with a dispersion medium of a specific combination.

In addition, since cerium oxide is in the form of powder, there is a problem in that aggregation of powders occurs. However, in this embodiment, as the cerium oxide is provided in the form of a dispersion in which the cerium oxide is dispersed in a specific combination of the dispersion medium, aggregation of the cerium oxide may be prevented. Accordingly, the skin barrier strengthening, moisturizing skin, and/or fine dust blocking effect may be improved, and the feeling of use such as spreadability, skin adhesion, and softness may be improved.

The cerium oxide of this embodiment may have an average particle diameter of 80 to 170 nanometers (nm), specifically 90 to 160 nanometers, and more specifically, 100 to 150 nanometers. In particular, when the average particle diameter of the cerium oxide of this embodiment is 80 to 170 nanometers, the skin barrier strengthening, moisturizing skin, and/or fine dust blocking effect of the cerium oxide dispersion according to this embodiment may be more excellent.

The dispersion medium comprises an ester-based oil and a polyol. The cerium oxide dispersion of the present embodiment comprises both ester-based oil and polyol as a dispersion medium, thereby enhancing skin barrier, moisturizing skin, and/or blocking fine dust.

The ester-based oil may comprise at least one selected from the group consisting of benzoate-based oil, adipate-based oil, carbonate-based oil, and caprylate-based oil.

The benzoate-based oil may comprise C12 to C15 alkyl benzoate.

The adipate-based oil may comprise dibutyl adipate.

The carbonate-based oil may comprise dicaprylyl carbonate.

The caprylate-based oil may comprise coco-caprylate/caprate.

When the ester-based oil of this embodiment comprises at least one selected from the group consisting of benzoate-based oil, adipate-based oil, carbonate-based oil, and caprylate-based oil, the cerium oxide dispersion according to the present embodiment may have a more excellent effect of strengthening skin barrier, moisturizing skin, and/or blocking fine dust.

On the other hand, in the present embodiment, when other types of oils are comprised instead of ester-based oils comprising at least one selected from the group consisting of benzoate-based oil, adipate oil, carbonate-based oil, and caprate-based oil, effects of strengthening skin barrier, moisturizing skin, and/or blocking fine dust may be degraded.

For example, the other types of oils which replace the ester-based oils comprising at least one ester-based oil selected from the group consisting of benzoate-based oil, adipate-based oil, carbonate-based oil, and caprate-based oil, may be silicon-based oil and eicosan-based oil but are not limited to thereto.

The silicone-based oil may comprise a silicone-based oil having a chain structure, and/or a silicone-based oil having a ring structure. For example, the silicone-based oil of the chain structure may be dimethicone, and the silicone-based oil of the ring structure may be cyclohexasiloxane, but is not limited thereto.

The eicosane-based oil may comprise at least one selected from the group consisting of eicosane and isoeicosane but is not limited thereto.

The polyol is a compound having two or more hydroxyl groups (-OH), and may comprise all known polyhydric alcohols. For example, the polyol may comprise at least one selected from the group consisting of glycerin, propylene glycol, dipropylene glycol, butylene glycol, and propanediol, but is not limited thereto.

The dispersion medium of the present embodiment may comprise the ester-based oil and the polyol in a weight ratio of 1: 0.1 to 0.7.

Specifically, the dispersion medium of the present embodiment may comprise the ester-based oil and the polyol in a weight ratio of 1: 0.2 to 0.6.

More specifically, the dispersion medium of the present embodiment may comprise the ester-based oil and the polyol in a weight ratio of 1: 0.2 to 0.5.

In particular, when the dispersion medium of the present embodiment comprises an ester-based oil and a polyol in the weight ratio as described above, the cerium oxide dispersion according to the present embodiment may have a more excellent effect of strengthening skin barrier, moisturizing skin, and/or blocking fine dust.

The cerium oxide dispersion of the present embodiment comprises the cerium oxide and the dispersion medium as described above in a weight ratio of 1:1 to 3.

Specifically, the cerium oxide dispersion of the present embodiment may comprise the cerium oxide and the dispersion medium as described above in a weight ratio of 1:1 to 2.

More specifically, the cerium oxide dispersion of the present embodiment may comprise the cerium oxide and the dispersion medium as described above in a weight ratio of 1:1 to 1.5.

In particular, when the cerium oxide dispersion of the present embodiment comprises the cerium oxide and the dispersion medium in the weight ratio as described above, the cerium oxide dispersion according to the present embodiment may have remarkably excellent effects of strengthening skin barrier, moisturizing skin, and/or blocking fine dust.

The cerium oxide dispersion of the present embodiment may further comprise an additive comprising at least one selected from the group consisting of a known coating agent, a thickening agent, a fatty substance, an organic solvent, a solubilizer, a thickener, a gelling agent, an emollient, an antioxidant, a suspending agent, a stabilizing agent, a blowing agent, a fragrance, a surfactant, an emulsifier, a solubilizer, a filler, a sequestering agent, a chelating agent, a preservative, a vitamin, a wetting agent, dyes, a pigment, a hydrophilic or lipophilic active agent, a lipid vesicle, and purified water with the cerium oxide and the dispersion medium.

In addition, the present disclosure provides a cosmetic composition comprising a cerium oxide dispersion for strengthening the skin barrier, moisturizing the skin, and/or blocking fine dust.

In another embodiment of the present disclosure, the embodiment provides a cosmetic composition comprising a cerium oxide dispersion as an active ingredient, wherein the cerium oxide dispersion comprises: cerium oxide; and a dispersion medium comprising an ester-based oil and a polyol, in which the cerium oxide and the dispersion medium are comprised in a weight ratio of 1:1 to 3.

As the cosmetic composition of the present embodiment comprises the cerium oxide dispersion, the effects of strengthening the skin barrier, moisturizing the skin, and/or blocking fine dust may be implemented. In addition, since the cosmetic composition of the present embodiment comprises cerium oxide in the form of a dispersion, the feeling of use such as a skin application property and skin adhesion may be excellent.

The cosmetic composition of the present embodiment may comprise 0.1 to 30% by weight of the cerium oxide dispersion, specifically 1 to 20% by weight, more specifically 5 to 15% by weight based on the total weight of the cosmetic composition. When the cosmetic composition of the present embodiment comprises the cerium oxide dispersion in an amount of 0.1 to 30% by weight based on the total weight of the cosmetic composition, the cosmetic composition of this embodiment is easy to formulate and has a good feeling of use such as skin spreadability and skin adhesion, and can realize excellent skin barrier strengthening, skin moisturizing, and/or fine dust blocking effects.

If the cosmetic composition of the present embodiment comprises less than 0.1% by weight of the cerium oxide dispersion based on the total weight of the cosmetic composition, the degree of skin barrier strengthening, moisturizing skin, and/or fine dust blocking effects may be insignificant. On the other hand, when the cosmetic composition of the present embodiment comprises more than 30% by weight of the cerium oxide dispersion based on the total weight of the cosmetic composition, it is difficult to implement the formulation, the skin irritation is increased, and the feeling of use may be reduced.

The cosmetic composition of the present embodiment may further comprise an additive comprising at least one selected from the group consisting of a known coating agent, a thickening agent, a fatty substance, an organic solvent, a solubilizer, a thickener, a gelling agent, an emollient, an antioxidant, a suspending agent, a stabilizing agent, a blowing agent, a fragrance, a surfactant, an emulsifier, a solubilizer, a filler, a sequestering agent, a chelating agent, a preservative, a vitamin, a wetting agent, dyes, a pigment, a hydrophilic or lipophilic active agent, a lipid vesicle, and purified water with the cerium oxide and the dispersion medium.

The cosmetic composition of the present embodiment may comprise the cerium oxide dispersion solution and a known additive, and/or carrier to be formed in any one formulation selected from the group consisting of a softening lotion, a nourishing lotion, an astringent lotion, a skin lotion, an essence, a cream, a massage cream, a pack, a makeup base, BB cream, a foundation, a lipstick, a lip balm, a lip tint, a lip gloss, a sun cream, a sunscreen lotion, a sunscreen milk, a sunscreen stick, a cleansing foam, a cleansing cream, and cleansing water.

The cosmetic composition of the present embodiment may be formed in the form of an oil-in-water (O/W), water-in-oil (W/O), water-in-oil-in-water (W/O/W), and oil-in-water-in-oil (O/W/O) type emulsion form but is not limited thereto.

The cosmetic composition, comprising the cerium oxide dispersion of the present disclosure, comprises all of the above-described cerium oxide dispersion for skin barrier strengthening, moisturizing skin, and/or fine dust blocking of the present disclosure.

Hereinafter, a cerium oxide dispersion for skin barrier strengthening, moisturizing skin, and/or fine dust blocking of the present disclosure and a cosmetic composition comprising the same will be described in detail through Examples, Comparative Examples, and Experimental Examples. These examples are only for illustrating the present disclosure, and therefore, the scope of the present disclosure is not to be construed as being limited by these examples.

[Example]

Example 1

(1) Preparation of cerium oxide dispersion

A cerium oxide dispersion was prepared by mixing cerium oxide and a dispersion medium in the composition of Table 1 [unit: weight ratio] and then stirring it at room temperature. At this time, the cerium oxide and the dispersion medium were mixed to obtain a total of 100% by weight, and the cerium oxide had an average particle diameter of 100 to 150 nanometers (nm).

[Table 1]

|  | Example |
|---|---|
| Cerium oxide | 1 |
| Dispersion medium | 1.5 |
| o The dispersion medium is a mixture of ester oil and polyol in a weight ratio of 1:0.5<br>o Ester oil: C12 to C15 alkyl benzoate<br>o Polyol: Butylene Glycol | |

(2) Preparation of a cosmetic composition including the cerium oxide dispersion

A water-in-oil (W/O) type cosmetic composition, including the cerium oxide dispersion as shown in Table 1 by weight as the composition of Table 2 [unit: wt%] was prepared in accordance with a known emulsification method.
In more detail, the oil phase components disclosed in Table 2 were added to the ultra homogenizer (mixing reactor) and stirred at 2,500 rpm for about 4 minutes to form an oil phase part. A water-in-oil type cosmetic composition was prepared by adding the aqueous phase components disclosed in Table 2 to the oil phase part and stirring at 5,000 rpm for about 4 minutes.

[Table 2]

| Role | Component | Example 1 |
|---|---|---|
| Emollient | Ethylhexyl Palmitate | 8.0 |
|  | Dibutyl adipate | 5.0 |
| Thickener | Disteadimonium Hectorite | 2.0 |
| Emollient | Phenyltrimethicone | 5.0 |
| Emulsifier | Polyglyceryl-3 Polyricinoleate | 2.5 |
|  | Sorbitan isostearate | 1.0 |
| Emulsifying adjuvants | Magnesium sulfate | 0.8 |
| Cerium oxide dispersion | | 10.0 |
| Purified water | | to. 100 |

Examples 2 to 4

(1) Cerium oxide dispersion and (2) cosmetic composition were prepared in the same manner as in Example 1, except that a dispersion medium including ester-based oil and polyol was used according to Table 3 [unit: weight ratio].

[Table 3]

|  | Example 1 | Example 2 | Example 3 | Example 4 |
|---|---|---|---|---|
| C12 to C15 Alkylbenzoate | 1 | 1 | 1 | 1 |
| Polyol | 0.5 | 0.05 | 0.2 | 0.8 |

Comparative Example

Comparative Examples 1 to 2

Cerium oxide dispersion and cosmetic composition were prepared in the same manner as in Example 1, except that the cerium oxide and the dispersion medium were mixed in the composition of Table 4 [unit: weight ratio].

[Table 4]

|  | Example 1 | Example 2 | Example 3 |
|---|---|---|---|
| Cerium oxide | 1 | 1 | 1 |
| Dispersion medium | 1.5 | 0.5 | 3.5 |

Comparative Examples 3 to 6

(1) Cerium oxide dispersion and (2) cosmetic composition were prepared in the same manner as in Example 1, except that a dispersion medium including dimethicone and polyol was used according to Table 5 [unit: weight ratio].

[Table 5]

|  | Comparative Example 3 | Comparative Example 4 | Comparative Example 5 | Comparative Example 6 |
|---|---|---|---|---|
| Dimethicone (Oil) | 1 | 1 | 1 | 1 |
| Polyol | 0.05 | 0.2 | 0.5 | 0.8 |

Comparative Examples 7 to 10

(1) Cerium oxide dispersion and (2) cosmetic composition were prepared in the same manner as in Example 1, except that a dispersion medium including cyclohexasiloxane and polyol was used according to Table 6 [unit: weight ratio].

[Table 6]

|  | Comparative Example 7 | Comparative Example 8 | Comparative Example 9 | Comparative Example 10 |
|---|---|---|---|---|
| Cyclohexasiloxane (oil) | 1 | 1 | 1 | 1 |
| Polyol | 0.05 | 0.2 | 0.5 | 0.8 |

Comparative Examples 11 to 14

(1) Cerium oxide dispersion and (2) cosmetic composition were prepared in the same manner as in Example 1, except that a dispersion medium including isoeicosan and polyol was used according to Table 7 [unit: weight ratio].

[Table 7]

|  | Comparative Example 11 | Comparative Example 12 | Comparative Example 13 | Comparative Example 14 |
|---|---|---|---|---|
| Isoeicosan (oil) | 1 | 1 | 1 | 1 |
| Polyol | 0.05 | 0.2 | 0.5 | 0.8 |

[Experimental Example]

Experimental Example 1: Confirmation of the primary stimulation test

In accordance with [Guidelines for Evaluation of Cosmetics New Material], a human patch test of the cosmetic compositions according to Examples 1 to 4 and Comparative Examples 1 to 14 was conducted.

The human patch test was conducted using the IQ Chamber for a total of 33 test subjects, with an average age of 41 ± 10 who participated in the human application test and satisfies the selection and exclusion criteria.

First, 20 µl of each test substance (cosmetic composition) was dropped into the IQ Chamber. The patch was attached for 24 hours, 1 hour after patch removal, 24 hours after patch removal, 2 experts observed the degree of irritation according to the International Contact Dermatitis Research Group (ICDRG) .

At this time, when the skin irritation index is 0.00 to 0.25, it is determined as non-irritation, when it is 0.3 to 1.0, it is determined as mild irritation, when it is 1.0 to 2.5, it is determined as moderate irritation, and when it is 2.5 to 4.0, it is determined as severe irritation, and the results are shown in Table 8.

The skin irritation index was calculated according to Formula 1 below.

[Formula 1]

$$\text{The level of skin reaction} = \left( \frac{\sum_{i=1}^{n} Evaluation\ value}{n(The\ number\ of\ test\ subjects)} \right)_{1hr} + \left( \frac{\sum_{i=1}^{n} Evaluation\ value}{n(The\ number\ of\ test\ subjects)} \right)_{24hrs}$$

$$\text{Skin irritation index} = \frac{The\ level\ of\ skin\ reaction}{n(The\ number\ of\ evaluations)}$$

[Table 8]

|  | Stimulus degree |  | Stimulus degree |  | Stimulus degree |
|---|---|---|---|---|---|
| Example 1 | 0.01 | Comparative Example 1 | 0.15 | Comparative Example 8 | 0.45 |
| Example 2 | 0.01 | Comparative Example 2 | 0.42 | Comparative Example 9 | 0.40 |
| Example 3 | 0 | Comparative Example 3 | 0.35 | Comparative Example 10 | 0.75 |
| Example 4 | 0.01 | Comparative Example 4 | 0.25 | Comparative Example 11 | 0.12 |
|  |  | Comparative Example 5 | 0.21 | Comparative Example 12 | 0.10 |
|  |  | Comparative Example 6 | 0.40 | Comparative Example 13 | 0.07 |
|  |  | Comparative Example 7 | 0.58 | Comparative Example 14 | 0.30 |

Referring to Table 8, it can be seen that Examples 1 to 4 according to the present embodiment have a lower degree of skin irritation than Comparative Examples 1 to 14, not according to the present embodiment. That is, the cosmetic composition including the cerium oxide dispersion, according to the present embodiment, means that there is almost no skin irritation.

Experimental Example 2: Evaluation of skin barrier strengthening ability

In order to confirm the skin barrier strengthening effect, the synthesis ability of ceramide constituting the skin barrier was evaluated according to the following method.

The cerium oxide dispersion according to Example 1 was treated on a 6-well plate at different concentrations (50 ppm and 100 ppm), and keratinizing cells were cultured for 24 hours.

It was used with an untreated control group for evaluation, and after the reaction was completed, the supernatant was collected, the plate was washed with a phosphate buffer saline (PBS), and the supernatant was recovered, and the expression amount was confirmed through Real-time PCR according to the PureLink® RNA Mini Kit (Ambion by Life Technologies) method. The amount of ceramide produced in the untreated group was converted to 100%, and the cerium oxide dispersion according to Example 1 was analyzed in the treated group.

In the same manner, as in Example 1, the CerS3 gene expression levels of the cerium oxide dispersion according to Examples 2 to 4 and Comparative Examples 1 to 14 were evaluated, and the results are shown in Table 9 [unit: ppm/mL].

[Table 9]

|  | $EC_{50}$ |  | $EC_{50}$ |  | $EC_{50}$ |
|---|---|---|---|---|---|
| Example 1 | 1.2 | Comparative Example 1 | 8.0 | Comparative Example 8 | 18.7 |
| Example 2 | 5.0 | Comparative Example 2 | >10 | Comparative Example 9 | 8.0 |
| Example 3 | 3.2 | Comparative Example 3 | >10 | Comparative Example 10 | >10 |
| Example 4 | 5.0 | Comparative Example 4 | >10 | Comparative Example 11 | >10 |
|  |  | Comparative Example 5 | 8.0 | Comparative Example 12 | >10 |
|  |  | Comparative Example 6 | >10 | Comparative Example 13 | 8.0 |
|  |  | Comparative Example 7 | >10 | Comparative Example 14 | >10 |

AS shown in Table 9, for the embodiment according to the present disclosure, it can be seen that the overall ceramide synthesis is promoted compared to the comparative example, not in accordance with this disclosure.

In more detail, comparing Example 1 with Comparative Examples 1 to 2, it may be seen that when the cerium oxide dispersion includes cerium oxide and the dispersion medium in a weight ratio of 1:1 to 3, the synthesis of ceramide is further promoted.

In addition, comparing Examples 1 to 4, it can be seen that the synthesis of ceramide is further promoted when the dispersion medium of the cerium oxide dispersion includes ester-based oil, including at least one selected from the group consisting of benzoate-based oil, adipate-based oil, carbonate-based oil, and caprylate-based oil and polyol, in particular when the two components of ester-based oil and polyol are included in a weight ratio of 1: 0.1 to 0.7.

In addition, comparing Example 1 and Comparative Examples 3 to 14, when the ester-based oil includes at least one selected from the group consisting of benzoate-based oil, adipate-based oil, carbonate-based oil, and caprate-based oil, the synthesis of ceramide is further promoted.

Experimental Example 3: Surface potential evaluation

The surface potential of the cosmetic composition, including the cerium oxide dispersion according to Example 1, was measured, and the results are shown in Table 10 [unit: mV]. Table 10 describes the average value obtained by measuring the surface potential three times.

At this time, the experiment was performed with an electrostatic measuring device (KSD-2000), and the surface potential was measured by bringing the probe of the measuring device into contact with the surface of the object to be measured.

In addition, for comparison of the surface potential between the cosmetic composition and fine dust, the surface potentials of fine indoor dust and fine outdoor dust were measured and shown in Table 10, and surface potential measurement of materials corresponding to the fine dust such as carbon black, iron oxide, and test fine dust (PM10 Test Dust) was also performed.

In this case, the carbon black and iron oxide having an average particle size of 10 micrometers or less were used. In addition, in the fine test dust, a mixture of fine test dust including: a carbon component containing organic carbon and elemental carbon; an ionic component containing sulfate, nitrate, ammonium salts; and a mineral component were used.

According to the same method as in Example 1, the surface potentials of the cosmetic compositions according to Examples 2 to 4 and Comparative Examples 1 to 14 were measured, and the average value thereof is also described in Table 10.

[Table 10]

|  | Zeta Potential (mV) |  | Zeta Potential (mV) |
|---|---|---|---|
| Indoor dust | -33.5 | Comparative Example 1 | -21.4 |

(continued)

| | Zeta Potential (mV) | | Zeta Potential (mV) |
|---|---|---|---|
| Outdoor dust | -35.6 | Comparative Example 2 | -29.8 |
| Carbon black | -30.1 | Comparative Example 3 | -31.2 |
| Iron oxide | -31.2 | Comparative Example 4 | -32.1 |
| Fine test dust | -33.3 | Comparative Example 5 | -33.4 |
| Example 1 | -56.2 | Comparative Example 6 | -24.0 |
| Example 2 | -35.4 | Comparative Example 7 | -25.5 |
| Example 3 | -48.1 | Comparative Example 8 | -27.5 |
| Example 4 | -40.1 | Comparative Example 9 | -31.0 |
| | | Comparative Example 10 | -21.0 |
| | | Comparative Example 11 | -35.6 |
| | | Comparative Example 12 | -36.0 |
| | | Comparative Example 13 | -36.1 |
| | | Comparative Example 14 | -30.1 |

As shown in Table 10, it may be seen that the case of Example according to the present disclosure generally exhibits a higher surface potential than that of Comparative Example not according to the present disclosure.

That is, according to the present Example, since the cosmetic composition has a higher surface potential than indoor dust, outdoor dust, carbon black, iron oxide, and fine dust for testing, it is possible to prevent the fine dust from adhering to the skin surface by static mechanical force.

Experimental Example 4: Evaluation of fine dust desorption performance

After coating the cosmetic composition of Example 1 to a uniform thickness on beige artificial leather, it was dried naturally. At this time, the cosmetic composition was coated in an amount of 2.0 mg/cm$^2$ to an application area of 24 cm$^2$.

After that, carbon black, iron oxide, and fine test dust were uniformly sprayed and applied to the artificial leather in which the cosmetic composition coated on the surface in an amount of 50 $\mu$g/m$^3$, and then the convection circulation device was operated for about 5 minutes, and then the degree of dropping of carbon black, iron oxide, and fine test dust was analyzed as images using image-j [image analysis program].

Based on the analyzed image, the ability to prevent adsorption of fine dust was evaluated, and the average result of three times was shown in Table 11 [unit: point].

The fine dust adsorption prevention ability was expressed as about 1 to 10 points, and the closer the score is to 10, the easier it is to remove the carbon black, iron oxide, and fine test dust from the artificial leather in which the cosmetic composition coated on the surface. It means that the fine dust adsorption prevention ability is excellent.

On the other hand, the closer the score is to 1, the more difficult it is to remove the carbon black, iron oxide, and fine test dust from the artificial leather in which the cosmetic composition coated on the surface, which means that there is almost no ability to prevent adsorption of fine dust.

According to the same method as in Example 1, the fine dust desorption performance of the cosmetic compositions according to Examples 2 to 4 and Comparative Examples 1 to 14 was evaluated, and the results are also described in Table 11.

[Table 11]

| | Fine dust adsorption prevention ability | | Fine dust adsorption prevention ability |
|---|---|---|---|
| Example 1 | 9.6 | Comparative Example 1 | 3.0 |
| Example 2 | 5.7 | Comparative Example 2 | 3.5 |
| Example 3 | 7.5 | Comparative Example 3 | 1.1 |

(continued)

| | Fine dust adsorption prevention ability | | Fine dust adsorption prevention ability |
|---|---|---|---|
| Example 4 | 5.5 | Comparative Example 4 | 1.5 |
| | | Comparative Example 5 | 1.9 |
| | | Comparative Example 6 | 1.0 |
| | | Comparative Example 7 | 3.7 |
| | | Comparative Example 8 | 3.8 |
| | | Comparative Example 9 | 3.9 |
| | | Comparative Example 10 | 4.0 |
| | | Comparative Example 11 | 3.5 |
| | | Comparative Example 12 | 3.9 |
| | | Comparative Example 13 | 4.1 |
| | | Comparative Example 14 | 3.0 |

As shown in Table 11, it can be seen that in the case of Examples according to the present disclosure, the overall effect of preventing adsorption of fine dust is superior to that of Comparative Examples not according to the present disclosure.

Experimental Example 5: Evaluation of skin moisturizing effect

In a group of 30 women aged 20 to 40, the cosmetic composition of Example 1 was applied twice daily to the face and surrounding areas for 1 month.

At this time, before applying the cosmetic composition, the amount of skin moisture is measured using a moisture meter (Corneometer, CM820 courage Khazaka Electronic GmbH, Germany) in constant temperature and humidity conditions (22 $\pm$ 2°C, humidity 50 $\pm$ 5%) and set as a default value, and the skin moisture content was measured after 1 week, 2 weeks, and 5 weeks after the cosmetic composition was applied, and the results are shown in Table 12 [unit: %]. In this case, a group not containing inorganic powder was used as a control group.

In the same manner, as in Example 1, the skin moisture content of the cosmetic compositions according to Examples 2 to 4 and Comparative Examples 1 to 14 was evaluated, and the results are shown in Table 12.

[Table 12]

| | Before application | 1 week after application | 2 weeks after application | 5 weeks after application |
|---|---|---|---|---|
| Example 1 | 68 $\pm$ 10 | 77 $\pm$ 10 | 82 $\pm$ 10 | 88 $\pm$ 10 |
| Example 2 | | 70 $\pm$ 10 | 72 $\pm$ 10 | 74 $\pm$ 10 |
| Example 3 | | 75 $\pm$ 10 | 78 $\pm$ 10 | 82 $\pm$ 10 |
| Example 4 | | 68 $\pm$ 10 | 71 $\pm$ 10 | 74 $\pm$ 10 |

(continued)

| | Before application | 1 week after application | 2 weeks after application | 5 weeks after application |
|---|---|---|---|---|
| Comparative Example 1 | | 68 ± 10 | 68 ± 10 | 70 ± 10 |
| Comparative Example 2 | | 68 ± 10 | 68 ± 10 | 70 ± 10 |
| Comparative Example 3 | | 70 ± 10 | 72 ± 10 | 72 ± 10 |
| Comparative Example 4 | | 70 ± 10 | 72 ± 10 | 72 ± 10 |
| Comparative Example 5 | | 70 ± 10 | 72 ± 10 | 72 ± 10 |
| Comparative Example 6 | | 68 ± 10 | 70 ± 10 | 72 ± 10 |
| Comparative Example 7 | | 68 ± 10 | 69 ± 10 | 70 ± 10 |
| Comparative Example 8 | | 68 ± 10 | 70 ± 10 | 72 ± 10 |
| Comparative Example 9 | | 68 ± 10 | 70 ± 10 | 72 ± 10 |
| Comparative Example 10 | | 68 ± 10 | 68 ± 10 | 70 ± 10 |
| Comparative Example 11 | | 68 ± 10 | 69 ± 10 | 70 ± 10 |
| Comparative Example 12 | | 68 ± 10 | 70 ± 10 | 72 ± 10 |
| Comparative Example 13 | | 68 ± 10 | 70 ± 10 | 72 ± 10 |
| Comparative Example 14 | | 68 ± 10 | 68 ± 10 | 70 ± 10 |

As shown in Table 12, in the case of the embodiment according to the present disclosure, it may be seen that the overall skin moisturizing effect is superior to that of the comparative example not according to the present disclosure.

Experimental Example 6: Evaluation of transepidermal water loss

After applying the cosmetic composition according to Example 1, the change in transepidermal water loss (TEWL) before and after the test was measured.

First, in order to observe changes in facial skin, the amount of transepidermal water loss in a total of three areas of the forehead, right cheek, and right eye area was measured, and the average value of the total three areas was used as the amount of transepidermal water loss of the facial skin. In addition, in order to observe changes in the neck skin, the amount of transepidermal water loss in a total of two areas on the front and side of the neck was measured, and the average value of the total two areas was used as the amount of transepidermal water loss in the neck skin. At this time, the amount of transepidermal water loss at each area was measured on the skin surface of each area using a probe of a transepidermal water loss measuring device.

In accordance with the above manner, the amount of transepidermal water loss of each area was repeatedly measured, and the average value thereof 5 times is shown in Table 13 [Unit: %] and Table 14 [Unit: %].

In addition, in the same manner, as in Example 1, the amount of transepidermal water loss of the cosmetic compositions according to Examples 2 to 4 and Comparative Examples 1 to 14 was measured, and the results are shown in Tables 13 and 14.

[Table 13]

| Face | Before application | After application |
|---|---|---|
| Example 1 | 59.1 | 58.9 |
| Example 2 | 60.1 | 62.5 |
| Example 3 | 59.1 | 59.0 |
| Example 4 | 60.1 | 63.5 |
| Comparative Example 1 | 61.0 | 65.1 |
| Comparative Example 2 | 61.0 | 66.7 |
| Comparative Example 3 | 61.2 | 62.6 |
| Comparative Example 4 | 61.2 | 62.6 |
| Comparative Example 5 | 60.5 | 63.0 |
| Comparative Example 6 | 60.5 | 65.5 |
| Comparative Example 7 | 59.1 | 65.9 |
| Comparative Example 8 | 59.1 | 64.1 |
| Comparative Example 9 | 60.1 | 68.6 |
| Comparative Example 10 | 60.1 | 67.8 |
| Comparative Example 11 | 55.1 | 66.3 |
| Comparative Example 12 | 60.1 | 65.4 |
| Comparative Example 13 | 59.1 | 64.0 |
| Comparative Example 14 | 55.1 | 68.1 |

[Table 14]

| Neck | Before application | After application |
|---|---|---|
| Example 1 | 61.0 | 59.1 |
| Example 2 | 60.0 | 63.0 |
| Example 3 | 55.1 | 54.3 |
| Example 4 | 61.3 | 65.3 |
| Comparative Example 1 | 61.0 | 66.7 |
| Comparative Example 2 | 61.0 | 68.1 |
| Comparative Example 3 | 62.8 | 69.6 |
| Comparative Example 4 | 61.0 | 66.0 |
| Comparative Example 5 | 61.0 | 65.4 |
| Comparative Example 6 | 62.8 | 70.0 |
| Comparative Example 7 | 58.1 | 67.8 |
| Comparative Example 8 | 59.1 | 66.0 |
| Comparative Example 9 | 59.1 | 65.4 |
| Comparative Example 10 | 58.1 | 69.0 |
| Comparative Example 11 | 59.1 | 65.0 |

(continued)

| Neck | Before application | After application |
|---|---|---|
| Comparative Example 12 | 60.1 | 65.1 |
| Comparative Example 13 | 60.1 | 64.8 |
| Comparative Example 14 | 59.1 | 70.1 |

As shown in Tables 13 and 14, it can be seen that in the case of the embodiment, according to the present disclosure, the skin barrier enhancing effect is generally superior to that of the comparative example not according to the present disclosure.

In view of the above contents, the cerium oxide dispersion and the cosmetic composition, including the same according to the present embodiment, may increase the moisture content of the skin, thereby implementing excellent skin moisturizing effects.

In addition, in view of the above contents, the cerium oxide dispersion and the cosmetic composition, including the same according to the present embodiment, may prevent the adsorption of fine dust, thereby implementing an excellent fine dust blocking function.

In addition, when taking the above contents into consideration, the cerium oxide dispersion and the cosmetic composition including the same according to this embodiment may promote the synthesis of ceramide and improving skin moisturizing power while preventing skin damage caused by fine dust, thereby implementing an excellent skin barrier reinforcement effect.

As described above, the description of the present disclosure is provided as an example, and it will be understood by a person skilled in the art that the present disclosure may be easily modified in other specific forms without changing the technical spirit or essential features of the present disclosure. Therefore, it should be understood that the embodiments described above are illustrative in all respects and not restrictive. For example, each component described as a single type may be implemented in a dispersed form, and likewise, components described as distributed may also be implemented in a combined form.

The scope of the present disclosure is indicated by the claims to be described below rather than the detailed description, and it should be interpreted that all changes or modifications derived from the meaning and scope of the claims and equivalent concepts are included in the scope of the present disclosure.

**Claims**

1. A cerium oxide dispersion for strengthening a skin barrier, the cerium oxide dispersion comprising:

    a cerium oxide; and
    a dispersion medium comprising an ester-based oil and a polyol,
    wherein the cerium oxide and the dispersion medium are comprised in a weight ratio of 1: 1 to 3.

2. A cerium oxide dispersion for moisturizing skin, the cerium oxide dispersion comprising:

    a cerium oxide; and
    a dispersion medium comprising an ester-based oil and a polyol,
    wherein the cerium oxide and the dispersion medium are comprised in a weight ratio of 1: 1 to 3.

3. A cerium oxide dispersion for blocking fine dust, the cerium oxide dispersion comprising:

    a cerium oxide; and
    a dispersion medium comprising an ester-based oil and a polyol,
    wherein the cerium oxide and the dispersion medium are comprised in a weight ratio of 1: 1 to 3.

4. The cerium oxide dispersion of any one of claims 1 to 3, wherein the dispersion medium comprises the ester-based oil and polyol in a weight ratio of 1: 0.1 to 0.7.

5. The cerium oxide dispersion of any one of claims 1 to 3, wherein the ester-based oil comprises at least one selected from the group consisting of benzoate-based oil, adipate-based oil, carbonate-based oil, and caprylate-based oil.

**6.** The cerium oxide dispersion of any one of claims 1 to 3, wherein the polyol comprises at least one selected from the group consisting of glycerin, propylene glycol, dipropylene glycol, butylene glycol, and propanediol.

**7.** A cosmetic composition for strengthening a skin barrier, moisturizing skin, or blocking fine dust, wherein the cosmetic composition comprises the cerium oxide dispersion of any one of claims 1 to 3 as an active ingredient.

**8.** The cosmetic composition of claim 7, wherein the cosmetic composition comprises 0.1 to 30% by weight of the cerium oxide dispersion with respect to the total weight of the cosmetic composition.

**9.** The cosmetic composition of claim 7, wherein the cosmetic composition comprises any one formulation selected from the group consisting of a soft lotion, a nourishing lotion, an astringent lotion, a skin lotion, an essence, a cream, a massage cream, a pack, a makeup base, BB cream, a foundation, a lipstick, a lip balm, a lip tint, a lip gloss, a sun cream, a sunscreen lotion, a sunscreen milk, a sunscreen stick, a cleansing foam, a cleansing cream, and cleansing water.

## INTERNATIONAL SEARCH REPORT

| International application No. |
| --- |
| **PCT/KR2020/010627** |

**A.    CLASSIFICATION OF SUBJECT MATTER**

*A61K 8/19*(2006.01)i; *A61K 8/37*(2006.01)i; *A61K 8/34*(2006.01)i; *A61K 8/04*(2006.01)i; *A61Q 19/00*(2006.01)i

According to International Patent Classification (IPC) or to both national classification and IPC

**B.    FIELDS SEARCHED**

Minimum documentation searched (classification system followed by classification symbols)

A61K 8/19; A61K 31/352; A61K 31/366; A61K 7/00; A61K 7/02; A61K 8/04; A61K 8/06; A61K 8/27; A61K 8/29; A61K 8/37; A61K 8/34; A61Q 19/00

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 피부 장벽 강화(enhancing skin barrier), 보습(moisturizing), 미세 먼지 차단 (protecting microdust), 화장료(cosmetic), 세륨 옥사이드(cerium oxide; CeO2), 에스테르계 오일(ester oil), 폴리올(polyol)

**C.    DOCUMENTS CONSIDERED TO BE RELEVANT**

| Category* | Citation of document, with indication, where appropriate, of the relevant passages | Relevant to claim No. |
| --- | --- | --- |
| A | KR 10-2019-0062937 A (SOULBRAIN CO., LTD.) 07 June 2019. See claims 1-17; paragraphs [0035]-[0071] and [0122]-[0126]; and table 1. | 1-9 |
| A | KR 10-2019-0048093 A (SOULBRAIN CO., LTD.) 09 May 2019. See claims 1-9; and paragraphs [0040]-[0046] and [0096]. | 1-9 |
| A | JP 2003-306410 A (FANCL CORP.) 28 October 2003. See claims 1-6; and paragraphs [0009]-[0021]. | 1-9 |
| A | KR 10-2010-0055734 A (AMOREPACIFIC CORPORATION) 27 May 2010. See claim 1; and paragraphs [0013]-[0024]. | 1-9 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **30 November 2020** | **30 November 2020** |

| Name and mailing address of the ISA/KR | Authorized officer |
| --- | --- |
| **Korean Intellectual Property Office** **Government Complex Daejeon Building 4, 189, Cheongsa-ro, Seo-gu, Daejeon, Republic of Korea 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2020/010627**

C.     DOCUMENTS CONSIDERED TO BE RELEVANT

| Category* | Citation of document, with indication, where appropriate, of the relevant passages | Relevant to claim No. |
|---|---|---|
| A | KR 10-2018-0012909 A (COSMAX, INC.) 07 February 2018. See claims 1-6; paragraphs [0011]-[0049]; and table 1. | 1-9 |
| PX | KR 10-2107760 B1 (KOLMAR KOREA CO., LTD.) 07 May 2020. See claims 1-6 and 8-9. This document is a published earlier application that serves as a basis for claiming priority of the present international application. | 1-9 |

Form PCT/ISA/210 (second sheet) (July 2019)

<table>
<thead>
<tr><th colspan="2">INTERNATIONAL SEARCH REPORT<br>Information on patent family members</th><th colspan="2">International application No.<br>**PCT/KR2020/010627**</th></tr>
</thead>
</table>

| Patent document cited in search report | | Publication date (day/month/year) | Patent family member(s) | | Publication date (day/month/year) |
|---|---|---|---|---|---|
| KR | 10-2019-0062937 A | 07 June 2019 | US | 2019-0159977 A1 | 30 May 2019 |
| KR | 10-2019-0048093 A | 09 May 2019 | US | 2019-0125639 A1 | 02 May 2019 |
| JP | 2003-306410 A | 28 October 2003 | None | | |
| KR | 10-2010-0055734 A | 27 May 2010 | KR | 10-1538468 B1 | 23 July 2015 |
| KR | 10-2018-0012909 A | 07 February 2018 | KR | 10-2019783 B1 | 10 September 2019 |
| KR | 10-2107760 B1 | 07 May 2020 | None | | |

Form PCT/ISA/210 (patent family annex) (July 2019)

**EP 4 023 206 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 100435855